# DEMANDE DE BREVET EUROPEEN

(11) **EP 1 481 597 A1**
(43) Date de publication de la demande: **01.12.2004**
(21) Numéro de dépôt: 04291321.0
(22) Date de dépôt: 25.05.2004
(51) Int. Cl.: A23P 1/08, A23L 1/00

(54) **Procédé de dragéification dure**

(30) Priorité: 26.05.2003 FR 0306320
(71) Demandeur: Roquette Frères, 62136 Lestrem (FR)
(72) Inventeur: Ortiz de Zarate, Dominique, 59660 Merville (FR)
(74) Mandataire: Doressamy, Clarisse

(57) **Abrégé**

L'invention a pour objet un procédé de dragéification dure sans sucre permettant la création d'un revêtement dur à la surface d'un noyau et comportant au moins un cycle comprenant :
- une étape d'application d'un sirop de polyol de richesse supérieure à 80%,
- une étape de séchage des noyaux,
caractérisé en ce que ce cycle ne comprend pas de temps de pause entre l'étape d'application du sirop de polyol et l'étape de séchage des noyaux.

## Description

L'invention se rapporte à un nouveau procédé de dragéification dure. Plus précisément, l'invention a pour objet un procédé de dragéification dure sans sucre permettant de réduire considérablement la durée de fabrication et de limiter les phénomènes de collage des centres dragéifiés.

La dragéification dure est une opération unitaire employée dans bon nombre de domaines et notamment en confiserie ou en pharmacie. Elle peut concerner également l'industrie des additifs que sont les arômes, les édulcorants, les vitamines, les enzymes, les acides et les produits à base de plantes. Cette opération consiste à créer un revêtement dur à la surface de produits solides ou pulvérulents, afin de les protéger pour diverses raisons ou bien afin de les rendre attractifs visuellement ou gustativement.

La dragéification dure vise à obtenir une couche croustillante et sucrée, toujours très appréciée dans le cas de confiseries comme les chewing-gums.

L'enrobage du noyau est effectué dans une cuve tournant autour de son axe et appelée drageuse, à l'intérieur de laquelle se trouvent une pluralité de noyaux formant une masse en mouvement, à la surface de laquelle on répartit à l'état liquide la matière constitutive de la future enveloppe.

La dragéification nécessite toujours l'utilisation d'un sirop contenant des matières cristallisables. Le revêtement dur et cristallin s'obtient par application de ce sirop et évaporation de l'eau apportée par celui-ci. Cette application doit être répétée un très grand nombre de fois de manière à obtenir le taux de grossissage voulu. De plus, il a toujours été nécessaire jusqu'alors de respecter un temps de pause entre la fin de l'étape d'application du sirop et le séchage des dragées, de manière à bien répartir le sirop à la surface des noyaux durant le temps de pause (temps durant lequel aucun sirop et aucune poudre ne sont appliqués à la surface des noyaux et durant lequel aucun air de séchage n'est insufflé dans la drageuse en rotation) et ainsi obtenir une cristallisation homogène du sirop lors de l'évaporation de l'eau qu'il contient au cours de l'étape de séchage.

La dragéification dure sans sucre est connue depuis longtemps et décrite notamment dans le brevet EP 0 037 407 dont la Demanderesse est titulaire, qui concerne un procédé de dragéification au sorbitol. L'enrobage se fait par cycles successifs comprenant chacun une première phase d'addition de sirop de sorbitol de richesse supérieure à 80% en poids sur sec sur le lit de noyaux, une seconde phase (temps de pause) pendant laquelle on arrête l'addition tout en maintenant la rotation de la cuve, et une troisième phase au cours de laquelle l'enveloppe dont ont été enrobés les noyaux est séchée par soufflage d'air chaud et sec de manière à assurer l'évaporation de l'eau apportée par le sirop et donc la cristallisation du polyol appliqué. Cette technique, bien que satisfaisante en termes de qualité du produit fini, reste longue en pratique, et peut provoquer des problèmes de collage des noyaux entre-eux au cours du temps de pause.

Une autre méthode visant à améliorer l'état de la technique a été décrite dans le brevet EP-B1-0 625 311 B1 dont la Demanderesse est titulaire.

Cette méthode permet d'obtenir des revêtements extrêmement durs et croustillants sans avoir besoin de procéder lors de chaque cycle de dragéification à une étape de séchage par soufflage d'air. Elle consiste en l'application d'un sirop de polyols de richesse au moins égale à 90% en poids par rapport à sa matière sèche, suivie d'une étape de répartition de sirop (premier temps de pause), d'une étape d'application d'une poudre du même polyol et d'une étape de répartition de la poudre appliquée (second temps de pause). Un temps dé pause de quinze secondes à deux minutes est respecté après ajout du sirop de polyols et avant ajout de la poudre de polyol.

Désireuse d'améliorer encore l'état de la technique, la Demanderesse s'est donc attachée à réduire davantage les temps de fabrication tout en optimisant la qualité des produits finis et c'est après de longs travaux de recherche qu'elle a constaté que l'on pouvait, de façon surprenante et inattendue, obtenir des revêtements durs sans sucre en un temps de fabrication considérablement réduit, dès lors que l'on supprime le temps de pause suivant la fin de la distribution du sirop de polyol, suivi soit d'une étape de séchage, soit des étapes d'application d'une poudre, de la répartition de cette poudre (pause) et de séchage.

Contre toute attente, on obtient selon ce procédé une dragéification tout à fait satisfaisante sans générer de collage des noyaux entre eux en un temps de fabrication sensiblement raccourci. Le procédé conforme à l'invention permet de gagner quinze secondes à deux minutes par cycle, ce qui est tout à fait avantageux quand on sait que le nombre de cycles effectué est généralement supérieur à 40.

L'invention a donc pour objet un procédé de dragéification dure sans sucre permettant la création d'un revêtement dur à la surface d'un noyau et comportant au moins un cycle comprenant :
- une étape d'application d'un sirop de polyol de richesse supérieure à 80%,
- une étape de séchage des noyaux,
caractérisé en ce que ce cycle ne comprend pas de temps de pause entre l'étape d'application du sirop de polyol et l'étape de séchage des noyaux.

Selon un mode général de réalisation de l'invention, le procédé consiste à humidifier de façon homogène la surface des produits à dragéifier en mouvement dans une turbine de dragéification à l'aide dudit sirop de polyol. Les sirops de polyols concernés par la présente invention sont des sucre-alcools choisis dans le groupe constitué par le sorbitol, le maltitol, le xylitol, les mélanges mannitol-xylitol, l'érythritol, le lactitol, l'isomalt (quelles que soient les proportions entre 1-1 GPM et 1-6 GPS dans l'isomalt), et leurs mélanges.

On utilisera de préférence un sirop présentant une richesse en polyol d'au moins 80% en poids sur sec, de préférence d'au moins 90%, plus préférentiellement d'au moins 92% et encore plus préférentiellement d'au moins 95%. Le sirop de dragéification peut être préparé à partir de tous types de polyols commercialisés sous forme de poudre. On peut également utiliser des sirops prêts-à l'emploi tel que par exemple des sirops de sorbitol ou des sirops de maltitol de haute richesse commercialisés par la Demanderesse.

La matière sèche du sirop est de préférence comprise entre 60 et 85% en poids, plus préférentiellement comprise entre 65 et 80%, et mieux encore comprise entre 65 et 75%.

Le sirop de polyols mis en oeuvre est porté à une température inférieure à 100°C avant application. Dans un mode de réalisation avantageux, la température du sirop est inférieure de préférence à 90°C et plus préférentiellement encore inférieure à 70°C.

La température régnant dans le lit en mouvement de noyaux à enrober est maintenue à une valeur inférieure à 55°C, de préférence comprise entre 10 et 50°C, et plus préférentiellement encore entre 15 et 40°C.

Les moyens pour le maintien de la température au sein de la masse de noyaux en mouvement peuvent être constitués par un dispositif d'insufflation d'air chaud de température contrôlée.

L'épaisseur de l'enveloppe peut être choisie librement en fonction notamment du noyau à dragéifier ou des effets recherchés.

Il est possible d'ajouter au sirop de polyols à pulvériser divers additifs comme des colorants, des arômes. On peut également prévoir des agents liants tels que des gommes végétales, la carboxyméthyl cellulose et la gélatine ou des substances grasses telles que les mono et diglycérides. On peut également prévoir de mélanger divers polyols dans le sirop d'enrobage. Le sirop de dragéification pourra également contenir des pigments tels que le carbonate de calcium, l'oxyde de titane, ou un colorant alimentaire, ainsi que des édulcorants intenses tels que l'aspartame, l'acésulfame K, la saccharine, le sucralose, l'alitame, le néotame, la néohespéridine, la thaumatine, le cyclamate de sodium ou de calcium.

Selon un mode particulier de réalisation de l'invention, on utilisera un sirop de sorbitol de haute pureté comme notamment le NEOSORB® 70/02 commercialisé par la Demanderesse, d'une richesse en sorbitol sur sec supérieure à 95%, et d'une matière sèche de 70%. Le sirop de dragéification préparé à partir du NEOSORB® 70/02 pourra avantageusement comprendre 1,5 à 2% de gomme arabique, 1% de dioxyde de titane, avoir une matière sèche de 65% et une température de 45°C. Un tel sirop de dragéification pourra contenir des arômes, des édulcorants intenses et tout autre ingrédient nécessaire à la dragéification.

A titre d'exemple, la composition de la matière sèche du sirop de dragéification pourra être 97,5% de sorbitol, 1,5% de gomme arabique, et 1% de dioxyde de titane.

La température du lit de noyaux en mouvement est de préférence maintenue à une température comprise entre 20 et 40°C. Le nombre de cycles application de sirop-séchage est compris entre 10 et 100, et de préférence compris entre 30 et 60. De très bons résultats ont été obtenus en réalisant 55 cycles successifs.

Une variante du procédé selon l'invention consiste à ajouter après application du sirop une quantité de poudre de polyol de haute pureté, de préférence supérieure à 90% en poids, et plus préférentiellement encore supérieure à 95% en poids.

Selon cette variante, le procédé conforme à l'invention comprend au moins un cycle comportant :
- une étape d'application d'un sirop de polyol de richesse supérieure à 80%,
- une étape d'application d'une poudre de polyol de pureté supérieure à 90%, de préférence supérieure à 95% en poids,
- un temps de pause pour répartir ladite poudre,
- une étape de séchage des noyaux,
et est caractérisé en ce que ce cycle ne comprend pas de temps de pause entre l'étape d'application du sirop et l'étape d'application de la poudre.

En ce qui concerne la poudre de polyol, on peut utiliser par exemple les poudres vendues sous les appellations NEOSORB® pour le sorbitol, XYLISORB® pour le xylitol, PALATINIT® pour l'isomalt, MALTISORB® pour le maltitol, PEARLITOL® pour le mannitol et LACTY® pour le lactitol.

Après répartition de la poudre et éventuellement séchage de l'ensemble, un nouveau cycle humidification-apport de poudre peut être réalisé. Le procédé nécessite au moins une, mais de préférence au moins deux applications, sous forme pulvérulente, d'une poudre de polyol de haute pureté. Ces applications peuvent être réalisées lors d'un même cycle ou bien lors de cycles différents sachant qu'un cycle est défini dans la présente invention comme ne comprenant qu'une seule application du sirop de polyol.

Selon une autre variante du procédé selon l'invention, ladite poudre de polyol est constituée par le même polyol que celui présent majoritairement dans le sirop de dragéification. L'homme du métier pourra alors suivre les enseignements du brevet EP 625 311 B1 dont la Demanderesse est titulaire, en ce qui concerne les compositions et paramètres de travail, tout en mettant en oeuvre le procédé selon l'invention qui consiste à supprimer au moins un temps de pause après application du sirop, c'est à dire à distribuer immédiatement la poudre dès la fin de la distribution du sirop dans au moins un cycle de dragéification.

Il est à noter que les produits à dragéifier peuvent éventuellement être gommés préalablement selon les techniques classiques connues de l'homme du métier. Bien entendu, il est possible de procéder en final à un cirage classique en vue d'améliorer l'aspect des produits. On utilise dans ce cas par exemple des corps gras, des laques ou des cires.

Une autre variante de l'invention consiste à réaliser une dragéification multicouches, mettant en oeuvre plusieurs polyols différents.

Le procédé conforme à l'invention permet d'enrober tous types de produits, comme notamment des produits alimentaires tels que des confiseries, chewing gums, bubble gums, comprimés, lozenges, articles gélifiés, pâtes à mâcher, bonbons durs, produits chocolatés, des fruits secs tels que amandes, noisettes, des produits pharmaceutiques ou vétérinaires comme les pilules, tablettes, produits pour animaux, des produits diététiques tels que les granulés de plantes, des semences ou des graines, des poudres d'engrais agglomérées, des additifs à base d'enzymes ou de microorganismes comme les levures, des tablettes détergentes, des vitamines, des arômes, parfums, acides, édulcorants ou principes actifs divers.

La mise en oeuvre de l'invention telle que décrite ci-avant permet d'obtenir des produits dragéifiés de très bonne qualité, avec des temps de dragéification relativement courts et plus courts que les procédés de l'art antérieur.

L'invention sera mieux comprise à l'aide des exemples suivants qui se veulent illustratifs et non limitatifs.

### Exemple 1 : dragéification dure au sorbitol selon l'invention.

Matériel utilisé : drageuse DRIACOATER 1200

### 1. Composition du sirop d'enrobage (65% matière sèche -45°C)

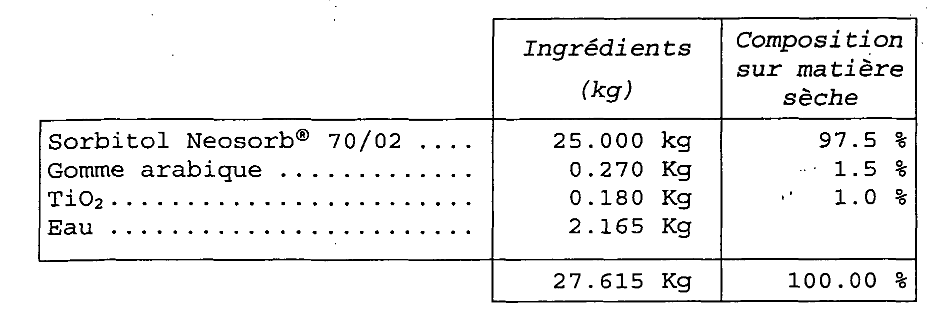

### 2. Séquences et paramètres de dragéification (60 Kg of chewing-gums)

### Phase 1 = dépoussiérage des centres et préchauffage

| Phase | 2 | 3 | 4 | 5 | 6 | 7 | 8 | 9 | 10 |
|---|---|---|---|---|---|---|---|---|---|
| Nombre de cycles | 5 | 5 | 5 | 10 | 10 | 11 | 3 | 5 | 1 |
| Quantité de sirop par cycle (Kg) | 0,4 | 0,6 | 0,7 | 0,9 | 1,1 | 1,2 | 0,8 | 0,4 | cire |
| **Pause (min)** | **0** | **0** | **0** | **0** | **0** | **0** | **1.0** | **2.0** | |
| Temps de séchage (min) | 3.5 | 3,5 | 3,5 | 3.5 | 4 | 4 | 3 | 1,5 | 5 |
| Température de séchage (°C) | 26 | 26 | 30 | 34 | 38 | 38 | 38 | 38 | 24 |
| Débit d'air chaud / m³/min | 12 | | | | | | | | |
| Vitesse de rotation drageuse (tours/min) | 12 | | | | | | | | |

Le temps total de la dragéification est de 231 minutes pour un taux de grossissage de 35%.

De l'arôme eucalyptus a été ajouté dans le sirop de dragéification.

### Exemple 2 : dragéification dure au sorbitol selon l'art antérieur.

### 1. Composition du sirop d'enrobage (65% matière sèche -45°C)

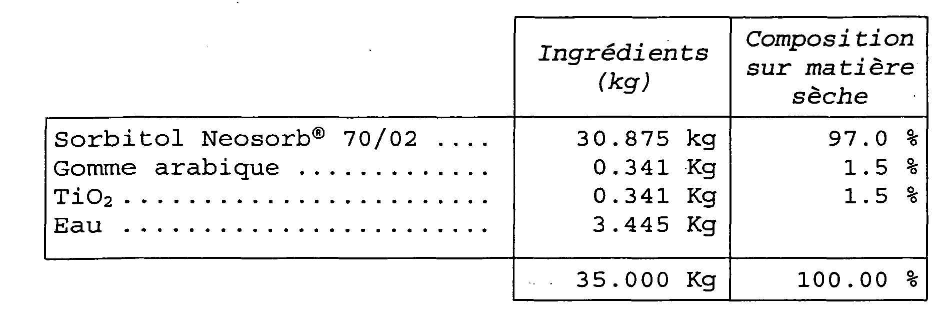

### 2. Séquences et paramètres de dragéification (50 Kg of chewing-gums)

### Phase 1 = dépoussiérage des centres et préchauffage

| Phase | 2 | 3 | 4 | 5 | 6 | 7 | 8 | 9 |
|---|---|---|---|---|---|---|---|---|
| Nombre de cycles | 4 | 9 | 6 | 12 | 19 | 4 | 5 | 1 |
| Qtité de sirop par cycle (Kg) | 0,3 | 0,4 | 0,5 | 0,6 | 0,7 | 0,7 | 0.3 | Cire |
| **Pause (min)** | **0,3** | **0,3** | **0,3** | **0.3** | **0.5** | **0.5** | **2.0** | **10.0** |
| Temps de séchage (min) | 4,5 | 4,0 | 4,0 | 3,0 | 3,5 | 3,0 | 1,0 | 5,0 |
| Température de séchage (°C) | 24 | 30 | 30 | 34 | 36 | 36 | 36 | 26 |
| Débit d'air chaud / m³ / min | 12 | | | | | | | |
| Vitesse de rotation drageuse (rpm) | 9 | 12 | 12 | 12 | 12 | 12 | 8 | 12 |

De l'arôme (mélange spearmint-menthol) a été ajouté à la surface des noyaux au cours des phases 6,7 et 8.

Le temps total de dragéification est de 246 minutes pour un taux de grossissage de 30% seulement.

Selon l'invention (exemple précédent), on obtient en moins de temps (231 minutes) un taux de grossissage supérieur (35%) .

Les produits sont comparables en termes de qualité organoleptique.

## Revendications

1. Procédé de dragéification dure sans sucre permettant la création d'un revêtement dur à la surface d'un noyau et comportant au moins un cycle comprenant :
- une étape d'application d'un sirop de polyol de richesse supérieure à 80%,
- une étape de séchage des noyaux,
**caractérisé en ce que** ce cycle ne comprend pas de temps de pause entre l'étape d'application du sirop de polyol et l'étape de séchage des noyaux.

2. Procédé selon la revendication 1, **caractérisé en ce que** ledit sirop de polyol comprend par rapport à sa matière sèche soluble au moins 80%, de préférence au moins 88% en poids de polyol.

3. Procédé selon l'une ou l'autre des revendications 1 et 2, **caractérisé en ce que** ledit sirop de polyol comprend un polyol choisi dans le groupe constitué par le sorbitol, le maltitol, le xylitol, le mannitol, l'érythritol, le lactitol, l'isomalt, et leurs mélanges.

4. Procédé de dragéification dure comportant au moins un cycle comprenant :
- une étape d'application d'un sirop de polyol de richesse supérieure à 80%,
- une étape d'application d'une poudre de polyol de pureté supérieure à 90%, de préférence supérieure à 95% en poids,
- un temps de pause pour répartir ladite poudre,
- une étape de séchage des noyaux,
**caractérisé en ce que** ce cycle ne comprend pas de temps de pause entre l'étape d'application du sirop et l'étape d'application de la poudre.

5. Procédé selon la revendication 4, **caractérisé en ce que** ledit sirop de polyol comprend par rapport à sa matière sèche soluble au moins 80%, de préférence au moins 88% en poids de polyol.

6. Procédé selon l'une ou l'autre des revendications 4 et 5, **caractérisé en ce que** ledit sirop de polyol comprend un polyol choisi dans le groupe constitué par le sorbitol, le maltitol, le mannitol, le xylitol, l'érythritol, le lactitol, l'isomalt, et leurs mélanges.

7. Procédé de dragéification dure selon l'une quelconque des revendications 4 à 6, **caractérisé en ce que** ladite poudre est constituée par le même polyol que celui présent majoritairement dans le sirop de dragéification.

8. Procédé selon l'une quelconque des revendications 1 à 7, **caractérisé en ce que** le produit à dragéifier est un produit alimentaire, vétérinaire ou pharmaceutique, diététique, une semence ou une graine, une poudre d'engrais ou encore un additif à base d'enzymes, de microorganismes, de vitamines, d'arômes, de parfums, d'acides, d'édulcorants ou de principes actifs.
